# EUROPEAN PATENT SPECIFICATION

(11) **EP 1 327 146 B1**
(45) Date of publication and mention of the grant of the patent: **14.09.2005**
(21) Application number: 01985752.3
(22) Date of filing: 26.09.2001
(51) Int. Cl.: G01N 33/543

(54) **COMPOUNDS WITH A BRANCHED LINKER**
SUBSTANZEN MIT VERZWEIGTEN LINKERMOLEKÜLEN
COMPOSES A LIAISON RAMIFIEE

(30) Priority: 29.09.2000 DE 10048417
(43) Date of publication of application: 16.07.2003
(73) Proprietor: Roche Diagnostics GmbH, 68305 Mannheim (DE); F.HOFFMANN-LA ROCHE AG, 4070 Basel (CH)
(72) Inventor: ANDRES, Herbert, 82377 Penzberg (DE); JOSEL, Hans-Peter, 82362 Weilheim (DE); HOESS, Eva, 81476 Muenchen (DE); HERRMANN, Rupert, 82362 Weilheim (DE); VON DER ELTZ, Herbert, 82362 Weilheim (DE)
(86) International application number: PCT/EP2001/011118
(87) International publication number: WO 2002/027315

(56) References cited:
- WO-A-99/53951
- US-A- 5 958 783
- US-A- 5 981 286
- KING H D ET AL: "MONOCLONAL ANTIBODY CONJUGATES OF DOXORUBICIN PREPARED WITH BRANCHED LINKERS: A NOVEL METHOD FOR INCREASING THE POTENCY OF DOXORUBICIN IMMUNOCONJUGATES" BIOCONJUGATE CHEMISTRY, AMERICAN CHEMICAL SOCIETY, WASHINGTON, US, vol. 10, no. 2, March 1999 (1999-03), pages 279-288, XP000804254 ISSN: 1043-1802

### SEQUENCE LISTING

<110> F. HOFFMANN-LA ROCHE AG
   Roche Diagnostics GmbH
<120> Compounds with a branched linker
<130> 19063WO
<140>
   <141>
<150> DE10048417.4
   <151> 2000-09-29
<160> 3
<170> PatentIn Ver. 2.1
<210> 1
   <211> 14
   <212> PRT
   <213> Artificial Sequence
<220>
   <223> Description of Artificial Sequence:peptide
<400> 1
   Asn Ser Trp Gly Cys Ala Phe Arg Gln Val Cys His Thr Thr
    1               5                  10
<210> 2
   <211> 18
   <212> PRT
   <213> Artificial Sequence
<220>
   <223> Description of Artificial Sequence:peptide
<220>
   <223> Xaa in position 3 denotes (Z) epsilon-amino caproic acid and Xaa in positions 2 and 4 denotes (U) beta-alanine
<400> 2
   Cys Xaa Xaa Xaa Asn Ser Trp Gly Cys Ala Phe Arg Gln Val Cys His
    1               5                  10                  15
   Thr Thr
<210> 3
   <211> 16
   <212> PRT
   <213> Artificial Sequence
<220>
   <223> Description of Artificial Sequence:peptide
<400> 3
   Leu Gly Ile Trp Gly Cys Ser Gly Lys Leu Ile Cys Thr Thr Ala Val
    1               5                  10                  15

## Description

The present invention concerns new linkers and their use for producing conjugates for applications in diagnostic or therapeutic methods.

Conjugates comprising several binding groups or/and effector groups e.g. labeling or solid phase binding groups or toxins are often used in diagnostic or therapeutic methods. Such conjugates can be prepared by direct coupling or by using the bridge or linker structures known in the prior art. Interfering intramolecular and intermolecular interactions between the conjugate partners or other components are often disadvantageous for the properties of such conjugates.

In diagnostic tests these undesired intramolecular and intermolecular interactions often lead to an impairment of important assay parameters such as the dynamic range of the signal, signal-to-noise ratio, breadth of the measuring range, blank value, lower limit of detection and thus to a considerable impairment of the assay. In therapeutic procedures the interactions in turn result in a reduction of efficacy or of target specificity.

The use of linkers that are known in the prior art for the conjugation of luminescent metal complexes (EP-A-0 178 450, EP-A-0 580 979, WO 87/06706) for example leads to a worsening of the dynamic range of an assay. Other disadvantages of such conjugates are a high unspecific binding to proteins and high blank values. However, similar problems also occur with other labeling and solid phase binding groups.

WO 96/03409 and WO 96/03410 disclose that the introduction of free positive or/and negative charge carriers in the linkers that link the reactive coupling group of the metal complex to one of the ligands or the introduction of hydrophilic groups into these luminescent metal complexes reduces the unspecific adsorption of conjugates of these complexes and thus improves the test sensitivity as well as the stability and recovery of the conjugates in immunoassays. Moreover in some cases it is possible to achieve an increase in quantum yield.

Bredehorst, R., et al., Anal Biochem 193 (1991) 272-9 describe a trifunctional haptenfluorophore conjugate which contains the 21 amino acid residues of the insulin A chain molecule as a backbone. The insulin A chain thus working as a linker between the fluorescent and hapten groups is a linear linker and not a branched linker.

In recent investigations it was found that the use of hydrophilic or charged linkers according to WO 96/03409 or WO 96/03410 results in considerable advantages in test performance but even when using such complexes the blank value is considerably higher than the blank value of the system. Hence a further reduction of the blank value by reducing unspecific binding would be desirable. In addition unspecific intramolecular and intermolecular interactions between the labeling group and other test components should be reduced without adversely affecting the signal yield and the accessibility of the labeling group.

It was surprisingly found that the said disadvantages can be eliminated by using branched linkers with charged carriers or/and hydrophilic groups especially in the side chains. These branched linkers also result in improvements in other types of conjugates used in diagnostic or therapeutic methods or for screening purposes.

Hence a subject matter of the present invention is the use of a polyfunctional compound of the general formula (I):

Zₙ - Y - Xₘ (I)

in which Z denotes at least one reactive functional group or a binding group, X is a reactive functional group which is bound covalently to Z via a linker Y where the linker is a branched linker which has a molecular weight of ≥ 1000 Da and contains at least one charge carrier or/and at least one hydrophilic group, n is an integer from 1 to 10 and preferably from 1 to 4 and m is 1 or 2 and preferably 1, for the production of conjugates.

The group Z can occur once or several times and can in each case be independently a reactive functional group or a binding group. Examples of binding groups are labeling groups or effector groups. Effector groups are for example partners of a bioaffine binding pair which can specifically interact with the other partner of the bioaffine binding pair.

The labeling groups can be selected from any detectable known groups such as dyes, luminescent labeling groups such as chemiluminescent groups e.g. acridinium esters or dioxetanes or fluorescent dyes e.g. fluorescein, coumarin, rhodamine, oxazine, resorufin, cyanine and derivatives thereof. Other examples of labeling groups are luminescent metal complexes such as ruthenium or europium complexes, enzymes as used for CEDIA (Cloned Enzyme Donor Immunoassay, e.g. EP 0 061 888), microparticles or nanoparticles e.g. latex particles or metal sols, and radioisotopes.

In a preferred embodiment the labeling group is a luminescent metal complex and the compound has a structure of the general formula (II):

[M(L₁L₂L₃)]ₙ - Y - XₘA (II)

in which M is a divalent or trivalent metal cation selected from rare earth or transition metal ions, L₁, L₂ and L₃ are the same or different and denote ligands with at least two nitrogen-containing heterocycles in which L₁, L₂ and L₃ are bound to the metal cation via nitrogen atoms, X is a reactive functional group which is covalently bound to at least one of the ligands L₁, L₂ and L₃ via a linker Y, n is an integer from 1 to 10, preferably 1 to 4, m is 1 or 2 and preferably 1 and A denotes the counterion which may be required to equalize the charge.

The metal complex is preferably a luminescent metal complex i.e. a metal complex which undergoes a detectable luminescence reaction after appropriate excitation. The luminescence reaction can for example be detected by fluorescence or by electrochemiluminescence measurement. The metal cation in this complex is for example a transition metal or a rare earth metal. The metal is preferably ruthenium, osmium, rhenium, iridium, rhodium, platinum, indium, palladium, molybdenum, technetium, copper, chromium or tungsten. Ruthenium, iridium, rhenium, chromium and osmium are particularly preferred. Ruthenium is most preferred.

The ligands L₁, L₂ and L₃ are ligands with at least two nitrogen-containing heterocycles. Aromatic heterocycles such as bipyridyl, bipyrazyl, terpyridyl and phenanthrolyl are preferred. The ligands L₁, L₂ and L₃ are particularly preferably selected from bipyridine and phenanthroline ring systems.

The complex can additionally contain one or several counterions A to equalize the charge. Examples of suitable negatively charged counterions are halogenides, OH⁻, carbonate, alkylcarboxylate, e.g. trifluoroacetate, sulphate, hexafluorophosphate and tetrafluoroborate groups. Hexafluorophosphate, trifluoroacetate and tetrafluoroborate groups are particularly preferred. Examples of suitable positively charged counterions are monovalent cations such as alkaline metal and ammonium ions.

On the other hand the group Z can be an effector group which interacts specifically and preferably non-covalently with a binding partner. Examples of suitable binding partners are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, receptor/ligand e.g. steroid hormone receptor/steroid hormone in which one partner of the binding pair is the effector group and thus a component of compound (I).

In a further preferred embodiment Z can also be a therapeutically active substance e.g. a toxin or protoxin e.g. an anti-tumour substance.

The compounds (I) are used as linker molecules to prepare conjugates. In this process a binding partner and in particular a binding partner as stated above is coupled covalently to the at least one free functional group of the compound (I).

The resulting coupling product contains at least two, preferably different, binding groups which are linked together via the branched linker Y.

The reactive functional group X or Z of the compound (I) or of the complex (II) is a reactive group which can be coupled covalently to a biological substance. The group X is preferably an activated carboxylic acid group such as a carboxylic acid halogenide, a carboxylic acid anhydride, a carboxylic acid hydrazide, a carboxylic acid azide or an active ester e.g. an N-hydroxy-succinimide, a p-nitrophenyl, pentafluorophenyl, imidazolyl or N-hydroxybenzotriazolyl ester, an amine, a maleimide, a thiol or a photoactivatable group e.g. an azide. The compound can contain one or several functional groups X or Z which can be the same or different. X and Z are preferably different. If Z is a functional group, it preferably only occurs once. If Z is a binding group, it can be present several times e.g. up to 10 times. The functional groups or binding groups Z, respectively can be the same or different and be optionally blocked by protective groups. However, the total number of groups X plus Z is at least 2 i.e. the compound is at least a bifunctional compound, preferably at least a hetero-bifunctional compound. Appropriate active groups for hetero-bifunctional linkers are described in Aslam M., Dent A., Bioconjugation (1998) Mcmillan Reference Ltd., London, p 216-363.

The molecular weight of the linker is at least 1000 Da, because then the advantages of the linker become particularly apparent The molecular weight of the linker is preferably in the range of 1000 to 50,000 Da, particularly in the range of 1000 to 20,000 Da and most preferably in the range of 1000 to 10,000 Da.

The compound (I) and the metal complex (II) differ from the prior art in that the linker Y between X and Z is a branched linker with at least one charge carrier or/and at least one hydrophilic group. In the sense of the present invention the term "charge carrier" means a group which is present mainly in an ionic form at a pH value in the range 6 to 8. The linker preferably contains up to 70, particularly preferably 1 to 40 and most preferably 2 to 20 such charge carriers.

The linker particularly preferably contains at least one negative charge carrier. Examples of suitable negative charge carriers are phosphate, phosphonate, sulphinate, sulphonate, sulphate and carboxylate groups, carboxylate groups and phosphate groups being most preferred.

Examples of positive charge carriers are amino and mono-substituted or polysubstituted amino groups such as mono-, di- or trialkyl amino groups, in which alkyl denotes a straight-chained or branched alkyl residue with 1 to 6 C atoms or a cyclic alkyl residue with 3 to 6 C atoms. The positive charge carriers are particularly preferably selected from basic amino acids such as lysine or substituted amino acids such as diethyllysine, or dipropyllysine. Amines and substituted amines can also be used as electron donors for the detection of metal complexes by electrochemiluminescence.

The linkers can also contain uncharged hydrophilic groups as an alternative to or in addition to the charge carriers. Preferred examples of uncharged hydrophilic groups are ethylene oxide or polyethylene oxide groups with preferably at least three ethylene oxide units, sulphoxide, sulphone, carboxylic acid amide, carboxylic acid ester, phosphonic acid amide, phosphonic acid ester, phosphoric acid amide, phosphoric acid ester, sulphonic acid amide, sulphonic acid ester, sulphuric acid amide and sulphuric acid ester groups. The amide groups are preferably primary amide groups, particularly preferably carboxylic acid amide residues in amino acid side groups e.g. the amino acids asparagine and glutamine. The esters are preferably derived from hydrophilic alcohols, in particular C₁-C₃ alcohols or diols or triols.

In the sense of the present invention the term "branched" means that the linker contains a main chain between the groups Z and X and in addition one or several side chains starting from the main chain. The charge carriers and hydrophilic groups can be located in the main chain or/and in a side chain. If the linker according to the invention contains several groups Z and X, the main chain itself can already be branched. However, the linker in any case additionally contains one or several side chains which contain none of the groups Z and X. The number of side chains is preferably 1 to 10, particularly preferably 2 to 6 and most preferably 2 to 4.

In a preferred embodiment of the invention the linker contains a main chain which contains one or several uncharged hydrophilic groups as mentioned above in particular carboxylic acid amide groups or/and polyethylene glycol groups while there is at least one charge carrier in one or several of the side chains. In this case 1 to 10 charge carriers and in particular 1 to 5 charge carriers can for example be present per side chain. Alternatively the linker can also contain charge carriers in the main chain and uncharged hydrophilic groups in one or several side chains. Furthermore embodiments are also conceivable in which the main chain and the side chains contain uncharged hydrophilic groups as well as charge carriers.

In case the branched linker comprises groups other than X and Z, which might interfere with the intended coupling chemistry, e.g., like -COO⁻ groups or -NH₂ groups, appropriate protective groups, which are known to the skilled artisan are used during synthesis and/or coupling. Terminal -NH₂ groups in peptidic side chains preferably are inactivated, e.g., by acetylation or succinylation.

The length of the main chain of the linker is preferably 7 to 200 atoms, particularly preferably 7 to 100 atoms. The main chain is an alkylene chain modified by the incorporation of heteroatoms e.g. O atoms or amide groups and contains at least one branch site. The side chains formed at the branching site preferably having a length of 4 to 100 atoms.

The charge carriers are preferably located in the linker in such a manner that a H atom of an alkylene unit of the main chain or/and in a side chain is replaced by a group containing a charge carrier e.g. NH₃⁺ or CO₂⁻.

The branched linker which contains the free charge carriers or/and hydrophilic groups is preferably at least partially composed of aminocarboxylic acid units that are linked together by peptide bonds. In such a linker the branching points can be derived from polyfunctional aminocarboxylic acids which contain at least three functional groups e.g. amino or carboxylate groups such that one functional group is still present after incorporation into the main chain which can be used as the starting point for the synthesis of the side chain. The branches are particularly preferably generated with diaminocarboxylic acids such as lysine, ornithine, hydroxylysine etc.

The charge carriers of the branched linker can be preferably derived from free positively or/and negatively charged groups of polyfunctional amino-carboxylic acids which contain a total of at least three charged groups e.g. amino, carboxylate or phosphate groups such that after incorporation into the linker and the concomitant reaction of two of the charged groups, at least one free charge carrier is still present. For example the charge carriers can be derived from trifunctional aminocarboxylic acids which contain (a) an amino group and two carboxylate groups or (b) two amino groups and one carboxylate group. Examples of such trifunctional aminocarboxylic acids are lysine, ornithine, hydroxylysine, aspartic acid and glutamic acid, symmetric trifunctional carboxylic acids like those described in EP 0 618 192 or US 5,519,142. Alternatively one of the carboxylate groups in the trifunctional aminocarboxylic acids (a) can be replaced by a phosphate, sulphonate or sulphate group. An example of such a trifunctional amino acid is phosphoserine.

Alternatively the branched linker can also be composed at least partially of phosphate-sugar units e.g. a DNA backbone without nucleobases or composed of glyco-peptidic structures. Furthermore the linker can also be composed at least partially of saccharide units. In any case the side chain of the linker is preferably situated at a branch site of the main chain which is formed by a trifunctional unit and the length of a side chain is at least two of the building blocks used for the synthesis e.g. natural or synthetic amino acids or other components such as ethylene glycol.

Preferably, the branched hydrophilic linker is used in immunological procedures to reduce problems caused by non-specific binding.

It has been found advantageous to use non-naturally occurring amino acids and non-naturally occurring sequence motives, like e.g., the di-peptides UE or UQ to construct a linker molecule according to the present invention. This has proven especially advantageous in serological assays, i.e. in assays set up to detect antibodies in patient sera. Linkers with non-naturally occurring β-amino acids have proven rather stable against proteases, e.g., against proteases in serum or plasma, and therefore represent a further preferred embodiment of the present invention.

In one embodiment of the present invention the branched linker comprising a metal complex has the general formula (III): 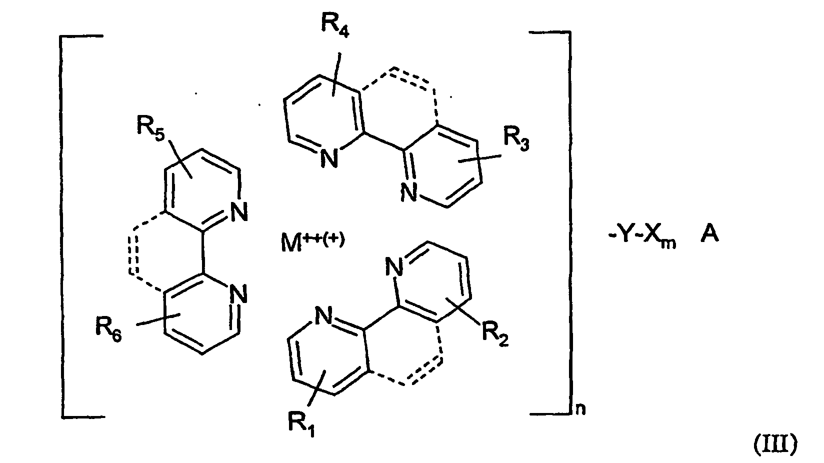 in which M, X, A, n and m are defined as above, R₁, R₂, R₃, R₄, R₅ and R₆ are the same or different and each denotes one or several substituents provided that X is bound to one of the ligands via one of the substituents R₁, R₂, R₃, R₄, R₅ or R₆ and the linker Y.

The ligands of the complex are optionally substituted phenanthroline or bipyridene systems depending on whether the groups denoted by the broken lines are present or not

The substituents R₁, R₂, R₃, R₄, R₅ and R₆ on the ligands are preferably hydrogen, C₁-C₅ alkyl, in particular C₁-C₃ alkyl, phenyl or a hydrophilic group as defined above provided they do not contain the linker Y.

In a particularly preferred embodiment the branched linker comprising a metal complex has the general formula (IIIa): 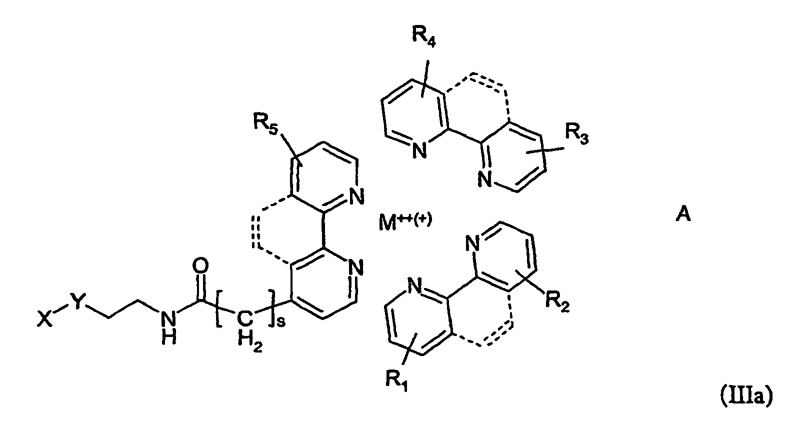 in which M, X and A are defined as above, R₁, R₂, R₃, R₄, R₅ are as defined above, s is an integer from 0 to 6 preferably from 1 to 4 and Y denotes the branched linker with free charge carriers or/and hydrophilic groups.

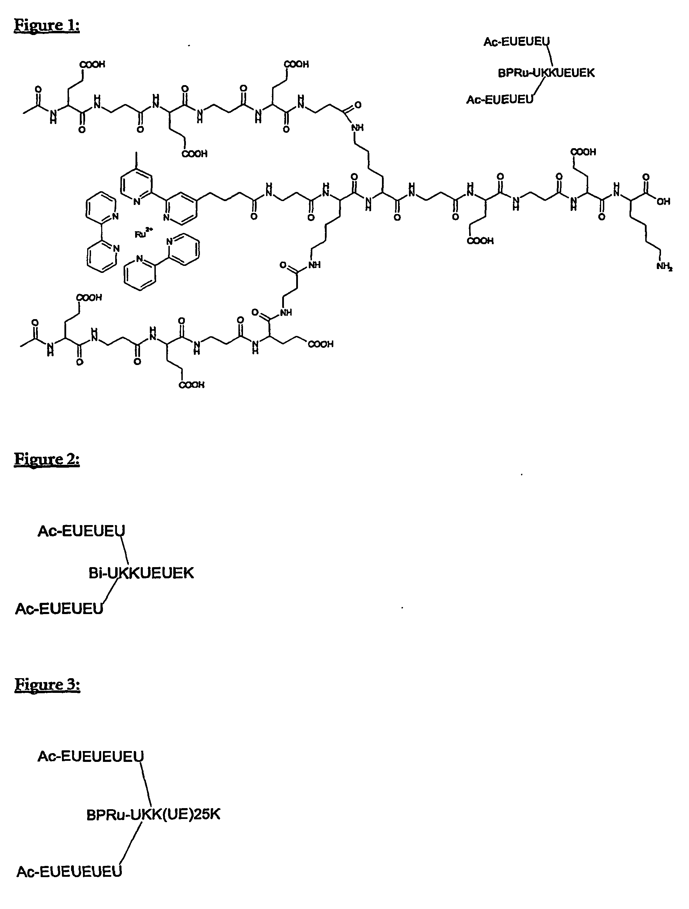

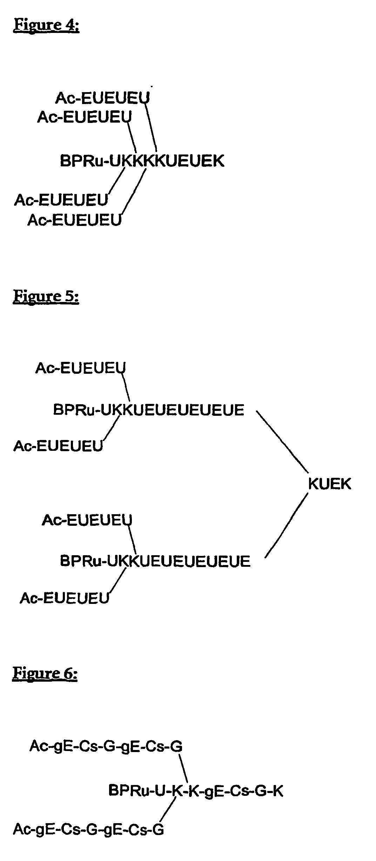

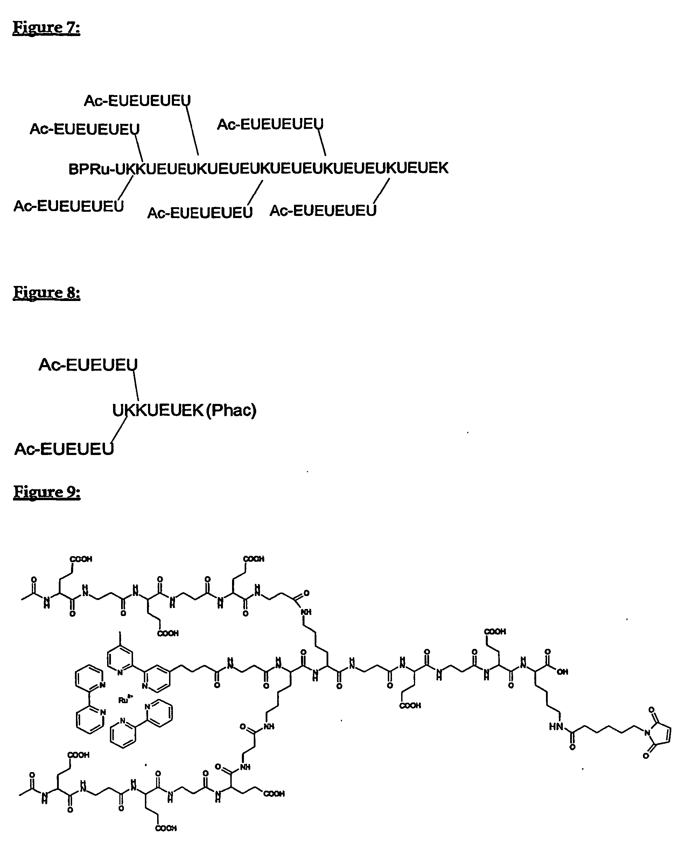

Examples of compounds of formula (I) with metal complexes as labeling groups or biotin as an effector group and the amino side group of lysine as the reactive functional group are shown in figs. 1-7. The branching points of the linker are formed by the trifunctional amino acid lysine which has two amino groups and one carboxylate group. One amino group and one carboxylate group are used to form peptide bonds in the main chain of the linker whereas the second amino group is used as the starting point for the synthesis of the side chain. The free charge carriers are formed from glutamic acid side chains. In Fig. 8 the lysine amino group is blocked by phenylacetyl (Phac). The blocking of one or several reactive groups in the linker by protective groups e.g. phenylacetyl or/and other protective groups compatible with the overall structure also enables labile binding groups to be introduced into the compound (I).

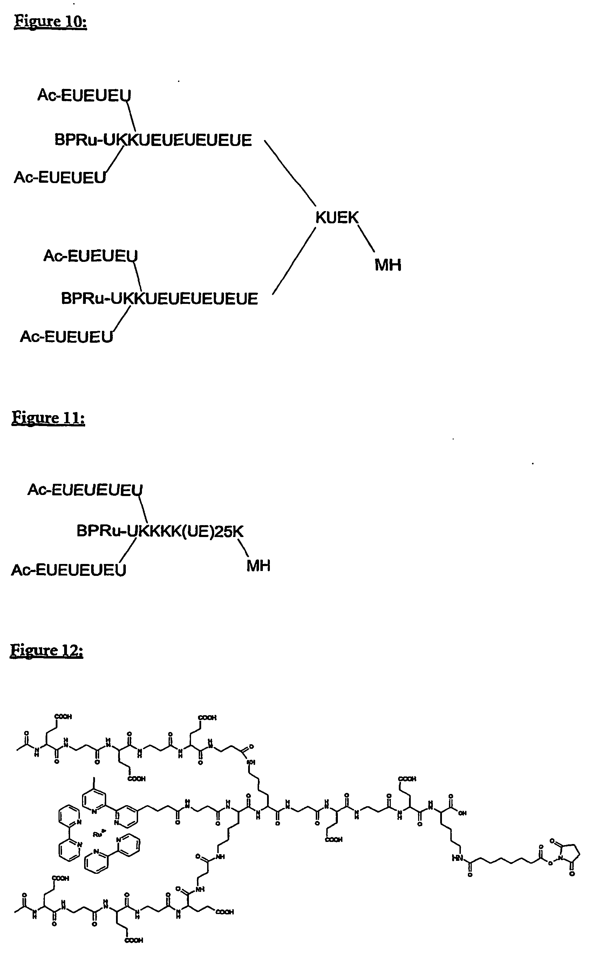

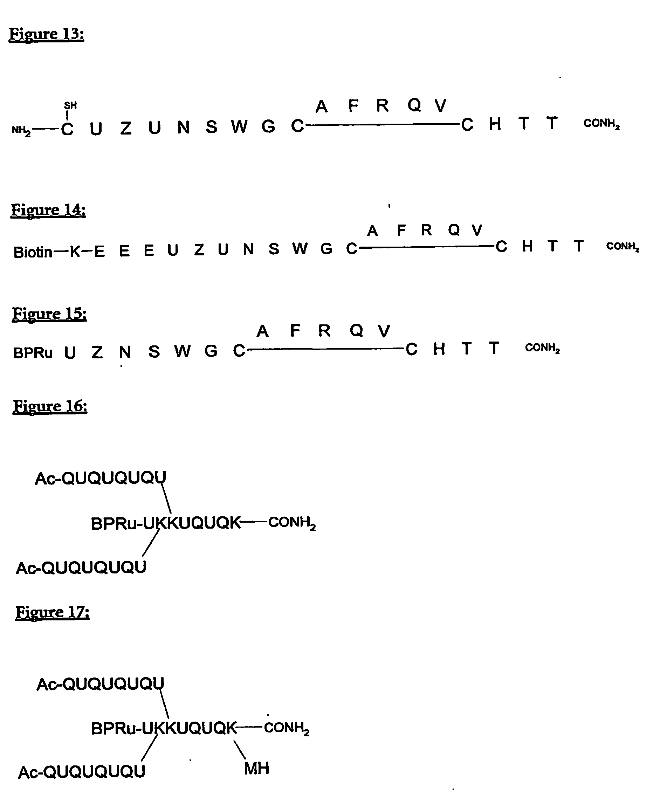

The reactive carboxylate group can for example be converted into an active ester by reaction with N-hydroxysuccinimide or disuccinimidyl suberate (DSS) (see Fig. 12). Alternatively the primary amino group can also be converted into a maleimide group by reaction with maleimidohexanoyl-N-hydroxysuccinimide ester (MHS) (see figs. 9 - 11).

The preparation of the compounds according to the invention is described in detail in the following using metal complexes as an example. Other compounds that for example contain biotin or peptide antigens as the effector group can be prepared in an analogous manner.

The synthesis of a charged and branched linker on a labeling group or solid phase binding group such as the N-heterocyclic ligand of a metal complex can be carried out as a coupling reaction in solution by coupling an optionally partially protected aminocarboxylic acid to a reactive group of the ligand e.g. a carboxylic acid. This coupling stage can optionally be repeated until a branched linker of the desired length has been synthesized. In this process at least one polyfunctional aminocarboxylic acid is introduced which contains one or several charged side groups.

Subsequently the reactive group X is introduced and protective groups that may be present on the side groups of the aminocarboxylic acids are cleaved off. This synthesis of the ligand by successively coupling amino acids in solution can take place on a single ligand and also on a ligand that has already been bound to a metal complex as the starting material. A suitable starting material is for example a luminescent metal complex which contains a free carboxylate group. Such complexes are known from the above-mentioned documents and are also offered commercially for example by the Igen Inc. Company, Rockville, MD, USA.

On the other hand the branched linker can also be prepared by solid phase peptide synthesis. In a first embodiment of the solid phase synthesis, an amino acid is coupled via its carboxylate group to the solid phase support and then the desired linker is synthesized by successively coupling further amino acids. In this process at least one amino acid which contains a charged group as a side group e.g. an amino group or a carboxylate group and at least one amino acid which serves as the branching site and is optionally in a protected form are used to prepare a linker according to the invention. After completion of the desired linker sequence, an activated metal complex, e.g. an active ester, can be coupled to the free N-terminal amino group of the peptide bound to the solid phase. After cleavage from the solid phase the reactive group X can be coupled to the carboxy terminus of the peptide linker and protective groups that may be present are cleaved off.

In another mode of the solid phase synthesis an amino acid-metal complex conjugate which contains a protected amino group and a carboxylate group e.g. Fmoc-Lys(-Ru(bipyridyl)₃-OH) can be anchored to a solid phase by means of the free carboxylate group and a peptide linker can be synthesized after release of the blocked amino group. After completion of the desired linker sequence, the complex is cleaved from the solid phase to obtain a linker which contains at least the original carboxylate anchor group as the free charge carrier. The reactive group X can be coupled to the amino terminus of the resulting peptide linker.

In a further procedure of solid phase synthesis the branched linker sequence with charge carriers can also be synthesized directly on a selected peptide epitope.

A combination of the above-mentioned synthesis variants can also be used to prepare the compounds according to the invention. Amino acid-metal complex conjugates that are suitable for the solid phase synthesis of the complexes according to the invention with a charged linker are described in DE 44 30 998.8. Reference is herewith made to this disclosure.

A further subject matter of the present invention is a compound of the general formula (I) as defined above.

Yet a further subject matter of the present invention is a conjugate comprising at least one biological substance to which at least one compound (I) according to the invention is coupled. Examples of suitable biological substances are cells, viruses, subcellular particles, proteins, lipoproteins, glycoproteins, peptides, polypeptides, nucleic acids, peptidic nucleic acids (PNA), oligosaccharides, polysaccharides, lipopoly-saccharides, cellular metabolites, haptens, hormones, pharmacological substances, alkaloids, steroids, vitamins, amino acids and sugars.

The compound is coupled to the biological substance by means of a reactive functional group of the compound that can covalently couple to a functional group of the biological substance. If the functional group is an active ester, it can for example be coupled to free amino groups of the biological substance. If the functional group is a maleimide residue, it can for example be coupled to free SH groups of the biological substance.

In a particularly preferred embodiment of the present invention the compounds are coupled to peptides which preferably have a maximum length of 50 amino acids and particularly preferably a maximum of 30 amino acids. These peptides are preferably prepared by synthesizing a peptide of the desired amino acid sequence on a solid phase during which a) a solid phase binding group or/and a labeling group e.g. an activated metal complex, preferably a metal complex-active ester derivative is coupled to the N-terminal amino group of the peptide or/and b) an amino acid derivative which is coupled covalently to an effector or/and labeling group e.g. a hapten or metal complex is introduced during the synthesis in at least one position of the peptide. The coupling of the effector group or/and labeling group e.g. to the N-terminal amino acid of the peptide is preferably carried out before cleaving the peptide from the solid phase and before cleaving protective groups on reactive side groups of the amino acid derivatives used for the peptide synthesis.

The peptides preferably contain one or several immunologically reactive epitope regions. These epitope regions are preferably derived from pathogenic organisms, e.g. bacteria, viruses and protozoa or from autoimmune antigens. The epitope region is particularly preferably derived from viral antigens and corresponds to the amino acid sequences of HIVI, HIVII, HIVO or hepatitis C virus (HCV).

Further preferred examples of biological substances are biotin, toxins, protoxins, nucleic acids, antibodies or antibody fragments, polypeptide antigens i.e. immunologically reactive polypeptides or haptens i.e. organic molecules having a molecular weight of 150 to 2000, in particular molecules with a steroid backbone such as cardenolides, cardenolide glycosides (e.g. digoxin, digoxigenin), steroid alkaloids, sex hormones (e.g. progesterone), glucocorticoids etc. Other examples of haptens are prostaglandins, leucotreines, leuco-endiines, thromboxanes etc.

Yet a further subject matter of the present invention is the use of the compounds according to the invention or the conjugates according to the invention in a detection method e.g. in an immunological detection method or an nucleic acid hybridization method, in particular in a luminescence assay.

If a metal complex is used as the labeling group, it is preferably detected by electrochemiluminescence in which luminescent species are generated electrochemically on the surface of an electrode. Examples for carrying out luminescence assays with metal complexes of the prior art can be found in EP 0 580 979, WO 90/05301, WO 90/11511 and WO 92/14138. Reference is herewith made to the methods and devices for luminescence assays disclosed therein. The electrochemiluminescence assays are carried out in the presence of a solid phase which is preferably composed of microparticles, in particular of magnetic microparticles which are provided with a reactive coating e.g. with streptavidin. In this manner it is possible to detect immune or hybridization complexes containing a metal complex as the labeling group that are bound to the solid phase.

The electrochemiluminescence measurement is preferably carried out in the presence of a reducing agent for the metal complex e.g. an amine. Aliphatic amines are preferred and in particular primary, secondary and tertiary alkylamines whose alkyl groups each have 1 to 3 carbon atoms. Tripropylamine is particularly preferred. The amine can, however, also be an aromatic amine such as aniline or a heterocyclic amine. The reducing agent can already be integrated into the ligand sphere of the complex.

In addition a surface active agent e.g. a non-ionic agent such as an ethoxylated phenol may be present as an amplifier. Such substances are for example commercially available under the names Triton X100 or Triton N401.

On the other hand the luminescent metal complex can also be detected by measuring the fluorescence or time-resolved fluorescence in which the metal chelate is excited by irradiation with light of a suitable wavelength and the resulting fluorescence radiation is measured. Examples for carrying out fluorescence assays are to be found in EP 0 178 450 and EP 0 255 534. Reference is herewith made to this disclosure.

The principle described in detail above of using metal complexes with branched and charged or hydrophilic linkers can be applied in an analogous manner to other labeling groups or/and effector groups. Other preferred test formats in which the branched linkers can be used are homogeneous assays. Such assays are based for example on the measurement procedures known as CEDIA or FRET (fluorescence resonance energy transfer, cf. e.g. Pope, A. J., et al., Drug Discov Today 4 (1999) 350-362) such as time-resolved FRET.

Considerable advantages compared to known test formats can be achieved by using the branched linkers according to the invention. Thus, e.g., positively charged luminescent metal complexes can be better handled within a conjugate comprising a negatively charged branched linker. An improved solubility and thus a lower unspecific binding is generally found when the branched linkers are used in combination with hydrophobic labeling groups or/and biological substances. In many cases this can be used to increase the number of labeling groups and thus to increase signal yields. Furthermore the sterically demanding branched linkers prevent interactions between hydrophobic labeling groups and hydrophobic biological substances which ensures an improved accessibility of the labeling group.

The branched linkers according to the invention can be very advantageous in diagnostic methods by for example reducing the blank value, improving the dynamic range of the test, lowering the lower limit of detection, broadening the test range or/and improving the signal-to-noise ratio. A reduction of the dose of active substance or/and a reduction of side effects can be achieved in therapeutic applications.

Yet a further aspect of the present invention is that linkers that carry one or several charge carriers or/and one or several hydrophilic groups as defined above cause a large shift of the apparent molecular weight in chromatographic methods such as gel electrophoresis e.g. agarose gel electrophoresis, SDS gel electrophoresis, gel filtration, hydrophobic interaction chromatography and ion exchange chromatography. This effect occurs with the branched linkers described in the present application as well as with the linear linkers described in WO 96/03409. As a result of this shift in the apparent molecular weight, i.e. the linkers appear to have a higher molecular weight than is actually the case, they can be used to prepare conjugates with a defined stoichiometry and homogeneous composition. After the linker which for example carries a defined number of labeling groups or effector groups, has been coupled to a binding group e.g. a biomolecule, the reaction products of the preparation can be simply obtained by chromatographic methods according to their stoichiometry (e.g. one molecule of linker per binding group, two molecules of linker per binding group, three molecules of linker per binding group etc.) in the form of separate fractions.

The linker used to prepare a particular conjugate should in this case have an apparent molecular weight of preferably ≥ 20 %, particularly preferably of ≥ 30 % and most preferably of ≥ 40 % of the apparent molecular weight of the binding group in the same chromatographic separation system.

Furthermore reagent kits (linker plus labeling group(s) or effector group(s) plus binding group e.g. biomolecule to be labeled), a system (including a measuring device to detect the respective labeling group) and a composition containing a reagent of defined stoichiometry and functionality are provided.

A preferred example of such conjugates with a defined stoichiometry are mono-digoxigenylated Fab' antibody fragment conjugates.

The present invention is further elucidated by the following examples and figures.

The following examples, references, sequence listing and figures are provided to aid the understanding of the present invention, the true scope of which is set forth in the appended claims. It is understood that modifications can be made in the procedures set forth without departing from the spirit of the invention.

### Figures:

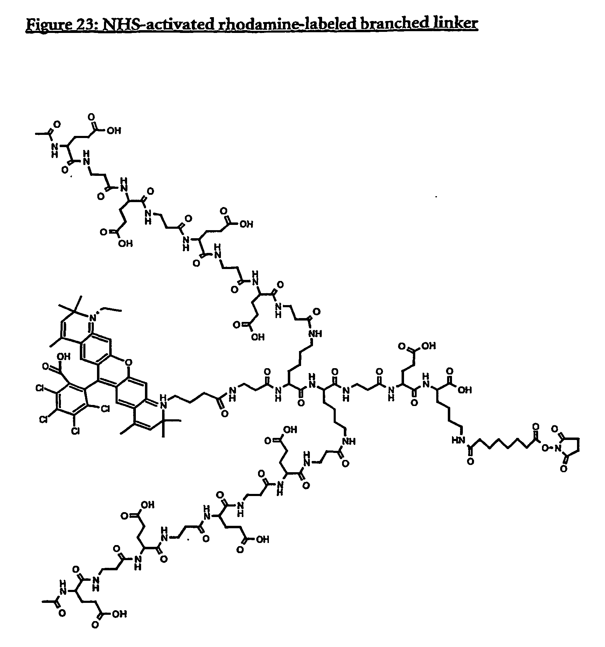

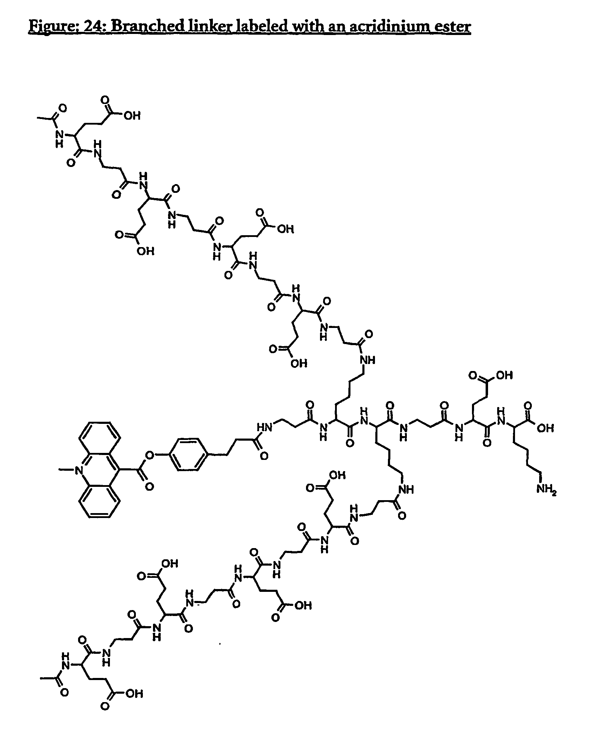

Fig. 1 to 12 and 16 to 20 as well as 23 to 25 show compounds according to the invention Fig. 13 to 15 show amino acid sequences of reference antigens, and Figures 21 and 22 show the solid-phase-bound branched linker (with and without the Mtt-protective group and with protective groups on the amino acid side chains) used to produce the conjugates of Fig. 23 to 25.

### Example 1: Preparation of branched linkers by means of solid phase peptide synthesis

The branched linkers were synthesized by means of fluorenylmethyloxycarbonyl-(Fmoc)-solid phase peptide synthesis on a batch peptide synthesizer e.g. from Applied Biosystems A433. In each case 4.0 equivalents of the amino acid derivative shown in table 1 were used for this.

**Table 1:**

| | |
|---|---|
| A | Fmoc-Ala-OH |
| C | Fmoc-Cys(Trt)-OH |
| D | Fmoc-Asp(OtBu)-OH |
| E | Fmoc-Glu(OtBu)-OH |
| gE | Fmoc-Glu-OtBu |
| F | Fmoc-Phe-OH |
| G | Fmoc-Gly-OH |
| H | Fmoc-His(Trt)-OH |
| I | Fmoc-Ile-OH |
| K1 | Fmoc-Lys(Boc)-OH |
| K2 | Fmoc-Lys(Fmoc)-OH |
| K3 | Fmoc-Lys(Dde)-OH |
| K4 | Fmoc-Lys(Alloc)-OH |
| K5 | Fmoc-Lys(PhAc)-OH |
| K6 | Fmoc-Lys-(label)-OH |
| K7 | Boc-Lys(Fmoc)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBU)-OH |
| U | Fmoc-β-alanine-OH |
| V | Fmoc-Val-OH |
| W | Fmoc-Trp-OH |
| Y | Fmoc-Tyr(tBU)-OH |
| Z | Fmoc-ε-amino caproic acid |
| Ps | Fmoc-Ser(PO(OBzl)OH)-OH |
| Cs | Fmoc-Cys(SO₃H)-OH |
| Mₜₜ-U | Mtt-β-alanine-OH |

The amino acids and amino acid derivatives were dissolved in N-methyl-pyrrolion. The peptide is synthesized on Wang resin (Wang, S. S., J Am Chem Soc 95 (1973) 1328-33). The resin is loaded with 0.2 to 0.4 mMol/g. The coupling reactions were carried out for 20 minutes using 4 equivalents dicyclohexylcarbodiimide and 4 equivalents N-hydroxybenzotriazole in dimethyl-formamide relative to the Fmoc-amino acid derivative in dimethylformamide as the reaction medium. The Fmoc group was cleaved after each step of the synthesis with 20 % piperidine in dimethylformamide for 20 min. The amount of resin was selected such that after the last branch, 4 equivalents Fmoc-amino acid relative to the amino groups are used. Fmoc-Lys(Fmoc)-OH is used for the branch and subsequent synthesis of two identical arms. The unsymmetric branches are achieved by amino acid derivatives with orthogonal side chain protective groups such as Fmoc-Lys(Dde) or Fmoc-Lys(Alloc). These orthogonal protective groups are cleaved on the resin by methods known in the literature (Bycroft, B. W., et al., J. Chem. Soc., Chem. Commun. 9 (1993) 778-9; Merzouk, A., et aL, Tetrahedron Letters 33 (1992) 477-80). Terminal amino groups on the solid phase are optionally acetylated or succinylated with acetic anhydride or succinic anhydride.

The hapten, label or functional group in those cases where the corresponding amino acid derivative is stable during the solid phase synthesis was already introduced on the resin e.g. on the N-terminal amino acid of the peptide.

The introduction of e.g. a metal chelate label was carried out via appropriate active ester derivatives at the free N-terminal amino group of the carrier-bound peptide. For this four equivalents ruthenium(bipyridyl)₃ complex (BPRu) per free primary amino function were activated with N-hydroxybenzotriazole/dicyclohexyl-carbodiimide and dissolved in a small amount of DMSO and this was added dropwise and stirred for 2 h at room temperature.

A hapten or a label can also be introduced at the C-terminus already during the solid phase synthesis by the direct incorporation of for example a metal chelate or biotin-coupled amino acid derivatives (described in WO 96/03409).

The peptide is released from the support and the acid-labile protective groups are cleaved with 20 ml tri-fluoroacetic acid, 0.5 ml ethanediol, 1 ml thioanisole, 1.5 g phenol and 1 ml water within 40 min at room temperature. Depending on the amino acid derivatives that are used, it is also possible to use cocktails containing fewer radical traps. 300 ml cooled diisopropyl ether was subsequently added to the reaction solution and was kept for 40 min at 0°C in order to completely precipitate the peptide. The precipitate was filtered, washed with diisopropyl ether and dissolved in a small amount of 50 % acetic acid and lyophilized. The crude material obtained was purified by means of preparative HPLC on Delta-PAK RP C18 (column 50 x 300 mm, 100 Å; 15 µ) over an appropriate gradient (eluant A: water, 0.1 % trifluoroacetic acid, eluant B: acetonitrile, 0.1 % trifluoroacetic acid) within ca. 120 min. The eluted material was identified by mass spectrometry.

Examples of such compounds prepared by solid phase synthesis are shown in figs. 1 to 7 and 16.

Alternatively the labeling group (label), the effector group (hapten) or the functional group can also be introduced after cleavage from the resin. For this it may be necessary to block other groups that should not be derivatized with a protective group which is stable during the solid phase peptide synthesis as well as during the cleavage (e.g. phenylacetyl (Phac)). The protective group can be removed enzymatically with PenG amidase (described in PCT/EP95/02921).

An example of a compound protected with Phac is shown in Fig. 8.

### Example 2: Introduction of a maleinimide function into a branched peptidic linker

In order to introduce the maleinimide function, a peptide according to example 1 was dissolved in 0.1 M potassium phosphate buffer pH 7.0, admixed with one equivalent maleinimidohexanoic acid N-hydroxysuccinimide ester (MH = maleinimidohexanoyl) in DMSO and stirred for 16 h at 25°C. The preparation was purified by preparative HPLC (see above). The identity of the eluted material was checked by means of mass spectrometry.

The compounds shown in Fig. 9 to 11 and 17 were prepared.

### Example 3: Introduction of N-hydroxysuccinimide ester groups into branched peptidic linkers

The experiment was carried out analogously to WO 96/03409, example 6.

The compound shown in Fig. 12 was prepared.

### Example 4: Use of metal complex-antigen conjugates with branched and charged linkers in immunological tests

A double antigen bridge test was carried out to detect specific antibodies to HIV. In this method the sample liquid was incubated with a ruthenium-labeled antigen and a biotinylated antigen for the antibody to be determined in the presence of a streptavidin-coated solid phase. The presence of anti-HN antibodies in the sample liquid is determined by measuring the label on the solid phase by means of electrochemiluminescence using the Elecsys® system. A partial sequence (SEQ ID NO: 1) of the gp36 protein of HIV2 is known to comprise antigenic epitopes as recognized by important antibodies in patient sera.

A peptide comprising SEQ ID NO: 1 extended at the N-terminus with an SH functional linker (SEQ ID NO: 2 and Fig. 13) was prepared as described in WO 96/03652. The full line between the two cysteines (C) of Fig. 13 shall indicate a -SS- cystine bridge.

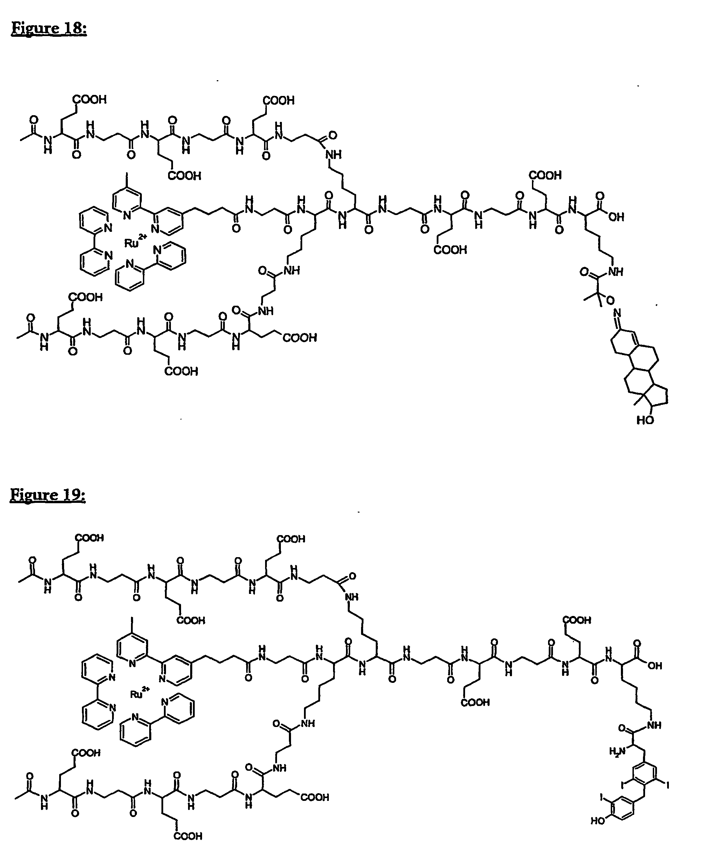

The peptide of SEQ ID NO: 2 (Fig. 13) was conjugated within 2 h at room temperature to the respective maleinimido-activated ruthenium linker in 0.1 mol/l potassium phosphate buffer pH 7 in order to derivatize the HIV peptide with maleinimido-activated ruthenium complexes. Non-reacted components were either separated by means of preparative HPLC or gel chromatography. The purified products were lyophilized.
- compound A:: BPRu linker from Fig.9 with gp36 antigen
- compound B:: BPRu linker from Fig. 10 with gp36 antigen
- compound C:: BPRu linker from Fig. 11 with gp36 antigen
- compound D:: BPRu linker from Fig. 17 with gp36 antigen

The conjugates comprising SEQ ID NO: 2 and one of the various linker variants (compounds A-D) were evaluated with the test format describe above. All evaluations were carried out with the same biotinylated peptide comprising the identical gp36 epitope of SEQ ID NO: 1 (Fig. 14) and at the same concentration. Labeled detection antigens were used in an equimolar concentration to the biotinylated capture antigen.

The conjugate shown in Fig. 15 and prepared as and described in PCT/EP95/02921 was used as a labeled reference antigen according to the prior art (comparison in tables 2 to 4).

The antigens according to the invention were used in equimolar amounts relative to the antigen of the prior art. The concentration was 0.018 nmol/l.

The result of the experiments with conjugates A and C compared to the compound from Fig. 15 is shown in ECL counts in table 2. It can be seen that considerably lower blank values with constant positive signals are only obtained by using the linker according to the invention which thus results in a better differentiation between positive signals and negative signals. These improved signal-to-noise ratios lead to an improvement of the measuring range.

**Table 2:**

| Experiment | Comparison | A | C |
|---|---|---|---|
| negative sample | 6014 | 2044 | 1975 |
| positive sample | 345247 | 484681 | 391007 |
| ratio positive/negative | 57.5 | 237.1 | 198 |

The result of the experiment with conjugate B in comparison to the compound of Fig. 15 is shown in ECL counts in table 3. It can be seen that the advantageous effects of the branched linker also enable the introduction of several labels without significantly increasing the blank value. It is also surprising that the positive signal is not quenched and even an increase in the measured counts is observed.

**Table 3:**

| Experiment | Comparison | B |
|---|---|---|
| negative sample | 6096 | 2366 |
| positive sample | 393197 | 765298 |
| ratio positive/negative | 64.5 | 323.5 |

Table 4 shows the result from the experiment with conjugate D compared to the compound of Fig. 15 in ECL counts. Surprisingly uncharged branched, hydrophilic linkers also have a positive effect on the blank value. The signal-to-noise ratio is improved.

**Table 4:**

| Experiment | Comparison | D |
|---|---|---|
| negative sample | 6718 | 2015 |
| positive sample | 553816 | 759947 |
| ratio positive/negative | 82.4 | 377.1 |

### Example 5: Preparation of antibody fragment conjugates

### 1. Description of the procedure

### Preparation of the Fab' from IgG

A monoclonal anti-Dig antibody was cleaved by pepsin to form F(ab')₂-fragments. After quantitative cleavage the pepsin was inactivated by increasing the pH and adding pepstatin. The F(ab')₂ was reduced by means of cysteamine to Fab' without prior purification. Cysteamine cleaves almost selectively the disulfide bridges in the hinge region. It was subsequently dialysed. This removes most of the Fc cleavage products generated by pepsin since they are small enough to pass through the pores of the dialysis tube (> 10,000 Dalton).

### Fab'-BPRU linker conjugate

The conjugate synthesis was carried out by reacting the Fab' with an excess of BPRu-linker-MH. In this process an SH group in the hinge region was mainly converted. Small amounts of polyruthenylated Fab' were formed as a side reaction most likely as a result of reduced intramolecular disulphide bridges in the light and in the Fd chain.

### Purification of the crude conjugate

The crude conjugate was purified by a molecular sieve. In this process the monoruthenylated material was separated from the polyruthenylated material.

### 2. Procedure

### Cleavage of the antibody to form F(ab')₂

The lyophilisate of the monoclonal antibody anti-DIG-M19.11 IgG was reconstituted with H₂O to obtain a concentration of 20 mg/ml. 20 µl 1 M citrate pH 3.5 were added per ml solution (final concentration citrate = 20 mM). The pH was adjusted with HCl to 3.60. It was filtered through a 0.45 µm filter. The concentration was determined at OD 280 nm (1 OD₂₈₀ₙₘ = 1.4 mg/ml). It was adjusted to 10 mg/ml with 20 mM citrate pH 3.60. The solution was heated in a water bath to 37°C. 100 µl pepsin solution (3 mg/ml) was added per ml antibody solution and incubated at 37°C in a water bath. After complete cleavage the reaction was stopped by increasing the pH value and adding pepstatin.

### Reduction to Fab'

52.6 µl 0.1 M dithiothreitol (DTT) was added per ml cleavage mixture and incubated for 30 min at 25°C in a water bath. The Fab' was dialysed against 0.1 M NaH₂PO₄/NaOH pH 6.5, 30 mM NaCl, 2 mM EDTA.

### Synthesis of the Fab'-BPRu-linker conjugate

The BPRu-linker-MH was dissolved in DMSO. The stoichiometry Fab':BPRu-linker-MH was 1:3 (mole/mole). The final concentration of Fab' in the mixture was 3.9 mg/ml. The maximum concentration of DMSO in the mixture was 10 %. The reaction time was 1 h at room temperature.

### Purification

The crude conjugate was concentrated 2-3-fold using an AMICON PM 10 and purified by means of Superdex 200 (buffer: 25 mM MOPS/NaOH pH 6.5, 50 mM NaCl, 10 % DMSO; applied amount: max 1.5 % of the gel bed, fractions: 0.5 % of the gel bed). The fractions containing the Fab'-BPRu-linker conjugate were pooled.

### Example 6: Use of metal complex-antibody fragment conjugates with branched and charged linkers in immunological tests

A double antigen bridge test was carried out to detect specific antibodies to HIV. In this method the sample liquid was incubated with a biotinylated antigen and a digoxigenin-labeled antigen to the antibody to be determined in the presence of a streptavidin-coated solid phase and anti-Dig-BPRu antibody. The presence of anti-HIV antibodies in the sample liquid was determined by determining the label bound via the double antigen bridge to the solid phase by electrochemiluminescence using the Elecsys® system.

A HIV peptide from the gp41 region of HIV1 (SEQ ID NO: 3), which was labeled at the N-terminus was used as the antigen. The preparation of the biotinylated and the digoxigenylated antigens is described in PCT/EP 95/02921. Two anti-Dig-BPRu conjugates were used for detection of ruthenylated gp41 peptide.
- Compound E:: anti-Dig-IgG-BPRu without linker
- Compound F:: anti-Dig-Fab'-BPRu with the linker of Fig. 9

Antigens were used in equimolar amounts at a concentration of 20 ng/ml.

The result of the experiments with compound E in comparison to compound F is shown in table 5. Digoxigenylated antigen and antibody conjugate (concentration 180 ng/ml) were pre-incubated. It can be seen that use of the linker according to the invention results in considerably lower blank values which is associated with a better differentiation between positive and negative signals. This is easily seen from the normalized values (table 5/2).

**Table 5:**

| Experiment [counts] | without BPRu conjugate = system blank value | compound E | compound F |
|---|---|---|---|
| negative sample | 441 | 2117 | 582 |
| positive sample 1 | 437 | 1215275 | 668410 |
| positive sample 2 | 453 | 49187 | 25819 |

**Table 5/2:**

| Experiment (normalized to negative sample) | without BPRu conjugate = system blank value | compound E | compound F |
|---|---|---|---|
| negative sample | 1.0 | 1.0 | 1.0 |
| positive sample 1 | 0.99 | 574.1 | 1148.5 |
| positive sample 2 | 1.03 | 23.1 | 44.4 |

In table 6 the test procedure was changed such that the magnetic beads with bound immune complexes were washed before adding the antibody conjugate (concentration 600 ng/ml). Also in this case the blank values were considerably lower and this was associated with an improved differentiation. The improved signal-to-noise ratio is especially evident from table 6/2, wherein the values have been normalized to the system blank as measured with the negative sample.

**Table 6**

| Experiment [counts] | without BPRu conjugate = system blank value | compound E | compound F |
|---|---|---|---|
| negative sample | 464 | 2771 | 758 |
| positive sample 1 | 453 | 1503690 | 686607 |
| positive sample 2 | 480 | 47040 | 27133 |

**Table 6/2:**

| Experiment pos./neg. sera | without BPRu conjugate = system blank value | compound E | compound F |
|---|---|---|---|
| negative sample | 1.0 | 1.0 | 1.0 |
| positive sample 1 | 0.98 | 592.6 | 905.8 |
| positive sample 2 | 1.03 | 17.0 | 35.8 |

Table 7 shows the unspecific binding of the antibody conjugate to the streptavidin solid phase. In this "test procedure" only buffer was used and neither the biotinylated nor the digoxigenylated antigens. The concentration of the ruthenylated antibody conjugate was 600 ng/ml. The linker according to the invention again exhibits improved blank values.

**Table 7:**

| Experiment [counts] | without BPRu conjugate = system blank value | compound E | compound F |
|---|---|---|---|
| negative sample | 333 | 3188 | 566 |
| buffer | 329 | 983 | 436 |

### Example 7: Preparation of further conjugates

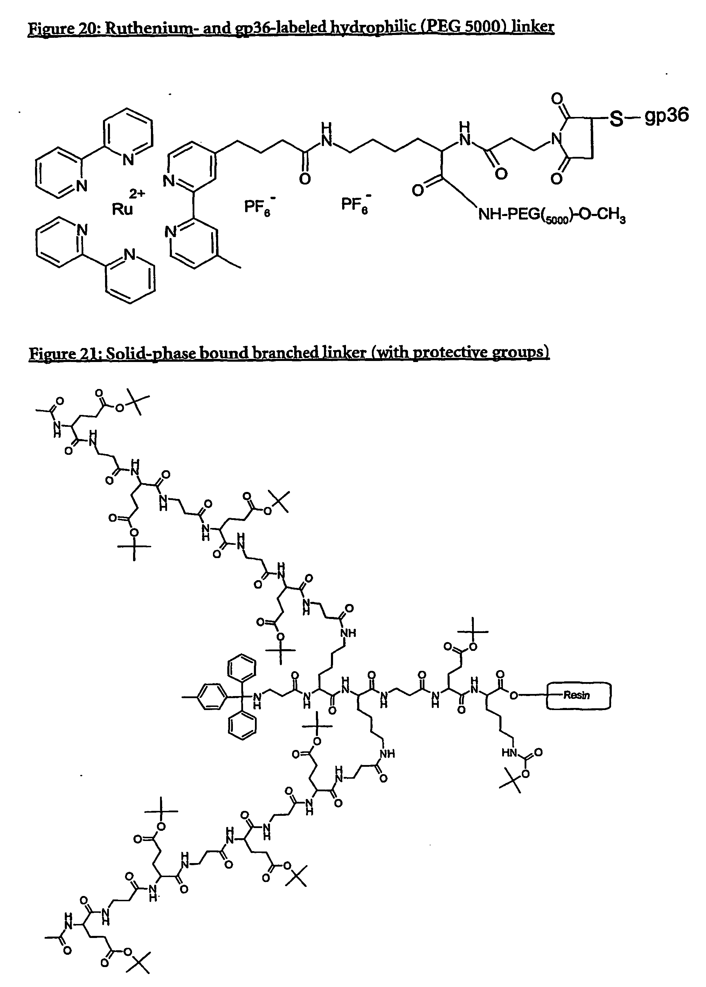

### 1. Synthesis of a testosterone derivative (Fig. 18)

8.5 mg BPRu-linker-NH₂ (Fig. 1) was dissolved in ca. 2 ml phosphate buffer pH 8.5 and 1.8 mg of the activated testosterone derivative (testosterone-3-dimethyl-carboxyoxime-NHS) dissolved in 2 ml dioxane was added dropwise. It was allowed to stir for 6 h at room temperature with the exclusion of light.

The crude product is purified by means of preparative HPLC. The molecular weight was confirmed by means of mass spectrometry (MALDI) as 3180.

### 2. Synthesis of a T3 derivative (Fig. 19)

The synthesis was carried out analogous to 7.1. MS-MALDI corresponded to the expected molecular weight.

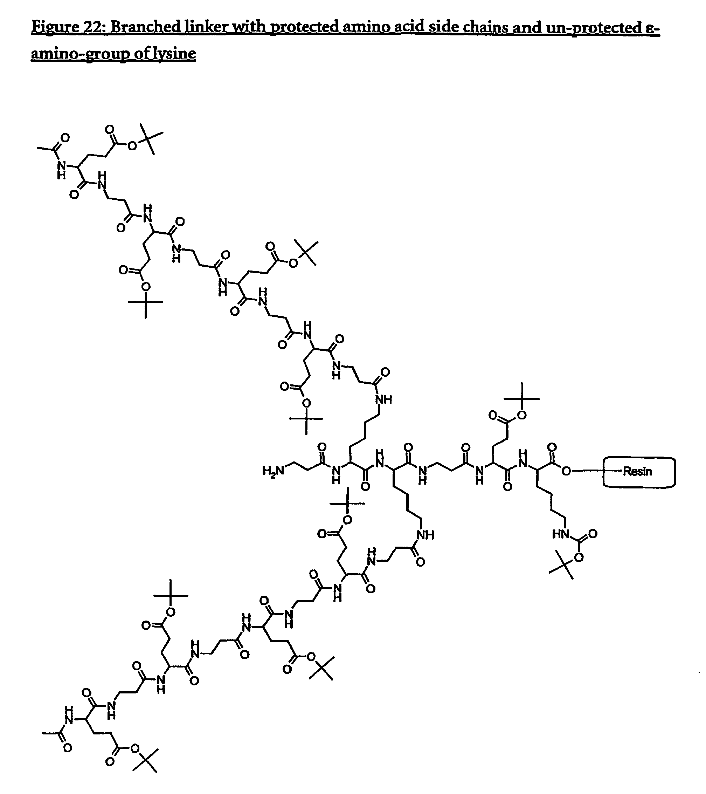

### 3. Synthesis of a PEG-Lys-MP-gp36 derivative (Fig. 20)

Starting with a lysine derivative of a ruthenium complex the free α-amino group of the lysine was reacted in the first step by conventional methods with maleinimido-propionic acid-(MP)-NHS ester. Then the carboxylic acid was activated by standard methods.

In the next step 3.64 mg of the active ester was reacted with 25.5 mg of an amino-modified polyethylene glycol H₂N-PEG-OCH₃-5000 (Shearwater) in 20 ml acetonitrile at room temperature. The product mixture was rotary evaporated and purified by means of gel chromatography (MALDI corresponded to the expected molecular weight).

The further coupling of the maleinimide to the gp36-peptide was carried out analogously to the already described method. The molecular weight determined with mass spectrometry corresponded to the expected molecular weight of 6990.

### 4. Synthesis of a fluorescence dye-labeled branched linker (Fig. 23)

The Mtt-protected branched peptidic linker (Fig. 21) is synthesized according to standard procedures.

The Mtt protecting group is cleaved off specifically according to the procedure described in Aletras A., et al., Int. J. Peptide Res. (1995) 45, 488 to yield the solid phase bound linker of Fig. 22.

50 mg of solid phase bound branched linker molecule (Fig. 22) with a single unprotected N-terminal amino group was suspended in 4 ml DMF. Afterwards 10 µl triethylamine and the activated fluorescence label (16 mg) was added and conjugated to the solid phase bound linker. (The synthesis of the activated label, the rhodamine-N-hydroxy succinimide ester, is described in DE 4137934.). The reaction mixture was stirred for 12 h at room temperature.

After cleavage from the solid phase under standard conditions (trifluoro acetic acid 95%), purification was performed with prep. HPLC.

Reaction of 16 mg of the intermediate with 18.5 mg disuccinimidyl suberate (DSS) in 10 ml DMF with 21 µl triethylamine for 5 h and standard purification led to 7 mg product (NHS-activated rhodamine-labeled branched linker as shown in Fig. 23). This was analyzed by MALDI-TOF and the molecular weight was found to correspond to the theoretical value.

### Example 8: Acridinium-labeled branched linker structures

### 1. Acridinium ester labeled branched linker

13 mg of the acridinium ester derivative (synthesis according to EP 82636) were added to 50 mg of solid phase bound linker (cf. Fig. 22) dissolved in 4 ml DMF and reacted as described in Example 7.4.

The acridinium-labeled linker was purified by prep. HPLC and lyophilised.

Product yield was found with 7 mg.

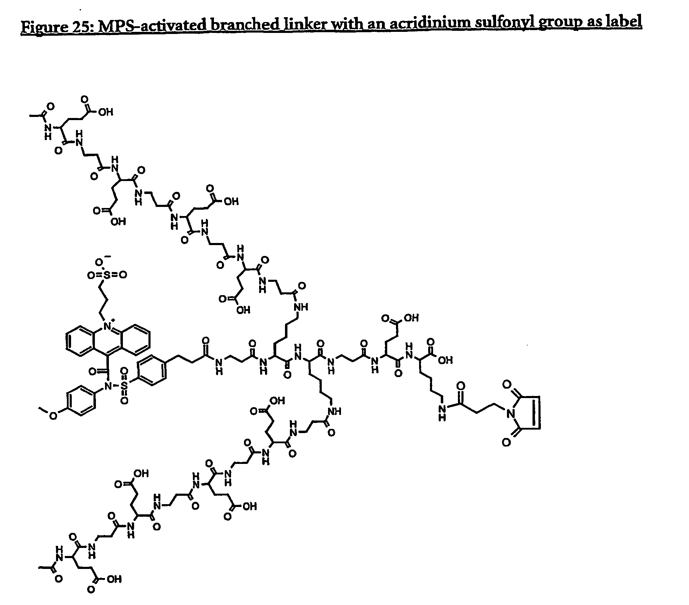

The product (cf. Fig. 24) was analyzed by MALDI-TOF and the molecular weight was found to correspond to the theoretical value.

### 2. Synthesis of acridinium sulfonyl labeled branched linker

The acridinium-labeled branched solid phase bound compound is synthesised as described in example 7.4 (synthesis of the acridinium sulfonamide see US 5,543,524). After cleavage from the solid phase and purification (see above) the free C-terminal amino group of the peptidic linker (4 mg linker) is reacted with 0,7 mg maleimidopropionyl-oxy succinimide ester (MPS) in 1 ml DMF (room temperature, 150 h) and purified by prep. HPLC.

Product yield was found with 2 mg.

The product (cf. Fig. 25) was analyzed by MALDI-TOF and the molecular weight was found to correspond to the theoretical value.

The MPS-activated acridinium sulfonamide labeled linker is conjugated to SH groups. In this example TSH (thyroid-stimulating hormone) was used and coupling performed according to a standard protocol (see Greg T. Hermanson, Bioconjugate Techniques, Academic Press, 1996, p. 456 ff).

In a comparison study using a two step immunoassay (with a washing step after the incubation of the sample with the first antibody) for TSH, the TSH-conjugate of this example was compared to a TSH-conjugate without linker. The TSH-concentrations are given as µIU/ml in table 8. Cal 1 and Cal 2 are commercial products, Roche Diagnostics order numbers TSH Cal Set: Id. Nr: 1731483, the samples marked Tris (100mM Tris, 1% BSA, 0,1% Thesit, 0,1% Oxaban, pH 7,4) comprise 0 and 50 µIU TSH per ml, respectively.

**Table 8:**

| | TSHµIU/ml | Signal-to-noise ratio (without linker) | Signal-to-noise ratio (with linker) |
|---|---|---|---|
| Cal1 | 0 | | |
| Cal2 | 1.43 | 2 | 7 |
| Tris 0 | 0 | | |
| Tris 50 | 50 | 17 | 127 |

It is obvious from table 8 that the TSH-conjugate comprising the branched linker according to the present invention shows a significant improvement with regard to signal-to-noise ratio as compared to a conjugate without such linker.

### List of References

Aletras A., et al., Int. J. Peptide Res. (1995) 45, 488
Aslam M., Dent A., Bioconjugation (1998) Mcmillan Reference Ltd., London, p 95 ff
Bredehorst, R., et al., Anal Biochem 193 (1991) 272-9
Bycroft, B. W., et al., J. Chem. Soc., Chem. Commun. 9 (1993) 778-9
Hermanson, G. T., Bioconjugate Techniques, Academic Press, 1996, p. 456 ff
Merzouk, A., et aL, Tetrahedron Letters 33 (1992) 477-80
Pope, A. J., et al., Drug Discov Today 4 (1999) 350-362
Wang, S. S., J Am Chem Soc 95 (1973) 1328-33

PCT/EP95/02921
DE 4137934
DE 44 30 998.8
EP 0 061 888
EP 0 178 450
EP 0 255 534
EP 0 580 979
EP 0 618 192
US 5,519,142
WO 87/06706
WO 90/05301
WO 90/11511
WO 92/14138
WO 96/03409
WO 96/03410
WO 96/03652

## Claims

1. Use of a compound of the general formula (I):
Zn - Y - Xm (I)
in which Z denotes at least one reactive functional group or a binding group and X is a reactive functional group which is bound covalently to Z via a linker Y wherein the linker is a branched linker which has a molecular weight of > 1000 Da and contains at least one charge carrier, n is an integer from 1 to 10 and m is 1 or 2, for the production of conjugates.

2. Use of a compound of the general formula (I):
Zn - Y - Xm (I)
in which Z denotes at least one reactive functional group or a binding group and X is a reactive functional group which is bound covalently to Z via a linker Y wherein the linker is a branched linker which has a molecular weight of > 1000 Da and contains at least one charge carrier and at least one hydrophilic group, n is an integer from 1 to 10 and m is 1 or 2, for the production of conjugates.

3. Use as claimed in claim 1 or 2, wherein the binding group is a labeling group or an effector group.

4. Use as claimed in any of claims 1 to 3, wherein the binding group is a labeling group selected from luminescent and fluorescent detection groups, enzymes, microparticles or nanoparticles and radio-isotopes

5. Use as claimed in claim 4, wherein the labeling group is metal chelate complex and the compound is of the general formula (II):
[M(L1L2L3)]n - Y - XmA (II)
in which
- M is a divalent or trivalent metal cation selected from rare earth or transition metal ions,
- L1, L2 and L3 are the same or different and denote ligands containing at least two nitrogen-containing heterocycles, L1, L2 and L3 being bound to the metal cation via nitrogen atoms,
- X is a reactive functional group which is covalently bound to at least one of the ligands L1, L2 and L3 via the branched linker Y,
- n is an integer from 1 to 10,
- m is 1 or 2 and
- A denotes the counterion which may be required to equalize the charge.

6. Use as claimed in one of the claims 1 to 5, wherein the binding group is an effector group selected from partners of specific bioaffine binding pairs.

7. Use as claimed in claim 6, wherein the effector group is selected from biotin and analogues thereof, streptavidin, avidin, antigens, haptens, antibodies, nucleic acids, nucleic acid analogues, sugars, lectins, receptors and receptor ligands.

8. Use as claimed in one of the claims 1 to 7, wherein the reactive functional group is a carboxylic acid, a carboxylic acid halogenide, a carboxylic acid anhydride, a carboxylic acid hydrazide, a carboxylic acid azide, an amine, an active ester, a maleimide, a thiol or a photoactivatable group.

9. Use as claimed in one of the claims 1 to 8, wherein the branched linker contains at least one negative charge carrier selected from the group consisting of phosphate, phosphonate, sulphinate, sulphonate, sulphate and carboxylate groups.

10. Use as claimed in claim 9, wherein the branched linker contains at least one carboxylate group or/and phosphate group.

11. Use as claimed in one of the claims 1 to 10, wherein the branched linker contains at least one positive charge carrier selected from amino groups and substituted amino groups.

12. Use as claimed in one of the claims 1 to 11, wherein the branched linker contains up to 70 charge carriers.

13. Use as claimed in claim 12, wherein the branched linker contains 1 to 40 charge carriers.

14. Use as claimed in one of the previous claims, wherein the branched linker contains at least one uncharged hydrophilic group selected from ethylene oxide, polyethylene oxide, sulphoxide, sulphone, carboxylic acid amide, carboxylic acid ester, phosphonic acid amide, phosphonic add ester, phosphoric acid amide, phosphoric acid ester, sulphonic acid amide, sulphonic acid ester, sulphuric add amide and sulphuric acid ester groups.

15. Use as claimed in claim 14, wherein at least one uncharged hydrophilic group is a primary carboxylic acid amide group.

16. Use as claimed in one of the previous claims, wherein the molecular weight of the linker is in the range from 1000 to 50,000 Da.

17. Use as claimed in one of the claims 1 to 16, wherein the branched linker is at least partially composed of aminocarboxylic acid units which are linked together by peptide bonds.

18. Use as claimed in claim 17, wherein the charge carriers are derived from polyfunctional aminocarboxylic acids which still contain at least one free charge carrier after incorporation into the linker.

19. Use as claimed in claim 17, wherein the hydrophilic groups are derived from polyfunctional aminocarboxylic acids which still contain at least one hydrophilic group after incorporation into the linker.

20. Use as claimed in claim 17, wherein the branching positions are derived from polyfunctional aminocarboxylic acids.

21. Use as claimed in claim 18, 19 or 20, wherein the polyfunctional aminocarboxylic acids are selected from lysine, ornithine, hydroxylysine, aspartic acid, glutamic acid, asparagine, glutamine, phosphoserine and synthetic trifunctional aminocarboxylic acids.

22. Compound of the general formula (I)
Zn - Y - Xm
wherein Z denotes at least one reactive functional group or a binding group and X is at least one reactive functional group which is covalently bound to Z via a linker Y where the linker is a branched linker that has a molecular weight of > 1000 Da and contains at least one charge carrier, n is an integer from 1 to 10 and m is 1 or 2.

23. Compound of the general formula (I)
Zn - Y - Xm
wherein Z denotes at least one reactive functional group or a binding group and X is at least one reactive functional group which is covalently bound to Z via a linker Y where the linker is a branched linker that has a molecular weight of > 1000 Da and contains at least one charge carrier and at least one hydrophilic group, n is an integer from 1 to 10 and m is 1 or 2.

24. Conjugate comprising at least one biological substance and at least one compound of the general formula (I) as claimed in claims 22 and 23.

25. Conjugate as claimed in claim 24, wherein the biological substance is an antibody or antibody fragment, a nucleic acid, a polypeptide antigen, an immunologically reactive peptide or a hapten.

26. Use of compounds as claimed in claims 22 or 23 or the conjugates as claimed in claim 24 or 25 in an immunological detection method or in a nucleic acid hybridization method.

27. Use as claimed in claim 26 in a luminescence method.

28. Use as claimed in claim 26 or 27 in an electrochemiluminescence method.

29. Use as claimed in claim 28 to improve the solubility of labeling groups or effector groups or of their conjugates.

## Patentansprüche

1. Verwendung einer Verbindung der allgemeinen Formel (I) :
Zn- Y - Xm (I),
wobei Z zumindest eine reaktive funktionelle Gruppe oder eine Bindungsgruppe bezeichnet und X eine reaktive funktionelle Gruppe ist, die über einen Linker Y kovalent an Z gebunden ist, wobei der Linker ein verzweigter Linker ist, der ein Molekulargewicht von > 1.000 Da aufweist und zumindest einen Ladungsträger enthält, n eine Ganzzahl von 1 bis 10 und m 1 oder 2 ist, zur Herstellung von Konjugaten.

2. Verwendung einer Verbindung der allgemeinen Formel (I) :
Zn- Y - Xm (I),
wobei Z zumindest eine reaktive funktionelle Gruppe oder eine Bindungsgruppe bezeichnet und X eine reaktive funktionelle Gruppe ist, die über einen Linker Y kovalent an Z gebunden ist, wobei der Linker ein verzweigter Linker ist, der ein Molekulargewicht von > 1.000 Da aufweist und zumindest einen Ladungsträger und zumindest eine hydrophile Gruppe enthält, n eine Ganzzahl von 1 bis 10 und m 1 oder 2 ist, zur Herstellung von Konjugaten.

3. Verwendung gemäß Anspruch 1 oder 2, wobei die Bindungsgruppe eine Markierungs- oder eine Effektorgruppe ist.

4. Verwendung gemäß einem der Ansprüche 1 bis 3, wobei die Bindungsgruppe eine Markierungsgruppe ist, ausgewählt aus lumineszenten oder fluoreszenten Detektionsgruppen, Enzymen, Mikropartikeln oder Nanopartikeln und Radioisotopen.

5. Verwendung gemäß Anspruch 4, wobei die Markierungsgruppe ein Metallchelatkomplex ist und die Verbindung von der allgemeinen Formel (II) ist:
[M(L1L2L3)]ₙ - Y - XmA (II), (II),
wobei
- M ein divalentes oder trivalentes Metallkation ist, das aus Seltenen Erden- oder Übergangsmetallionen ausgewählt ist,
- L1, L2 und L3 gleich oder unterschiedlich sind und Liganden kennzeichnen, die zumindest zwei Stickstoff enthaltende Heterozyklen enthalten, wobei L1, L2 und L3 über Stickstoffatome am Metallkation gebunden sind,
- X eine reaktive funktionelle Gruppe ist, die über den verzweigten Linker Y kovalent mit zumindest einem der Liganden L1, L2 und L3 verbunden ist,
- n eine Ganzzahl von 1 bis 10 ist,
- m 1 oder 2 ist und
- A das Gegenion **kennzeichnet**, das zum Ausgleichen der Ladung erforderlich sein mag.

6. Verwendung gemäß einem der Ansprüche 1 bis 5, wobei die Bindungsgruppe eine Effektorgruppe ist, die aus Partnern von spezifischen bioaffinen Bindungspaaren ausgewählt ist.

7. Verwendung gemäß Anspruch 6, wobei die Effektorgruppe ausgewählt ist aus Biotin und Analogen desselben, Streptavidin, Avidin, Antigenen, Haptenen, Antikörpern, Nukleinsäuren, Nukleinsäureanalogen, Zuckern, Lectinen, Rezeptoren und Rezeptorliganden.

8. Verwendung gemäß einem der Ansprüche 1 bis 7, wobei die reaktive funktionelle Gruppe eine Carbonsäure, ein Carbonsäurehalogenid, ein Carbonsäureanhydrid, ein Carbonsäurehydrazid, ein Carbonsäureazid, ein Amin, ein aktiver Ester, ein Maleimid, ein Thiol oder eine photoaktivierbare Gruppe ist.

9. Verwendung gemäß einem der Ansprüche 1 bis 8, wobei der verzweigte Linker zumindest einen negativen Ladungsträger enthält, der ausgewählt ist aus der Gruppe, die aus Phosphat-, Phosphonat-, Sulfinat-, Sulfonat-, Sulfat- und Carboxylatgruppen besteht.

10. Verwendung gemäß Anspruch 9, wobei der verzweigte Linker zumindest eine Carboxylatgruppe oder/und Phosphatgruppe enthält.

11. Verwendung gemäß einem der Ansprüche 1 bis 10, wobei der verzweigte Linker zumindest einen positiven Ladungsträger enthält, der ausgewählt ist aus Aminogruppen und substituierten Aminogruppen.

12. Verwendung gemäß einem der Ansprüche 1 bis 11, wobei der verzweigte Linker bis zu 70 Ladungsträger enthält.

13. Verwendung gemäß Anspruch 12, wobei der verzweigte Linker 1 bis 40 Ladungsträger enthält.

14. Verwendung gemäß einem der vorstehenden Ansprüche, wobei der verzweigte Linker zumindest eine ungeladene hydrophile Gruppe enthält, die ausgewählt ist aus Ethylenoxid-, Polyethylenoxid-, Sulfoxid-, Sulfon-, Carbonsäureamid-, Carbonsäureester-, Phosphonsäureamid-, Phosphonsäureester-, Phosphorsäureamid-, Phosphorsäureester-, Sulfonsäureamid-, Sulfonsäureester-, Schwefelsäureamid- und Schwefelsäureestergruppen.

15. Verwendung gemäß Anspruch 14, wobei zumindest eine ungeladene hydrophile Gruppe eine primäre Carbonsäureamidgruppe ist.

16. Verwendung gemäß einem der vorstehenden Ansprüche, wobei das Molekulargewicht des Linkers im Bereich von 1.000 bis 50.000 Da liegt.

17. Verwendung gemäß einem der Ansprüche 1 bis 16, wobei der verzweigte Linker zumindest teilweise aus Aminocarbonsäure-Einheiten besteht, die durch Peptidbindungen miteinander verknüpft sind.

18. Verwendung gemäß Anspruch 17, wobei die Ladungsträger aus polyfunktionellen Aminocarbonsäuren abgeleitet sind, die noch zumindest einen freien Ladungsträger nach Einbau in den Linker enthalten.

19. Verwendung gemäß Anspruch 17, wobei die hydrophilen Gruppen von polyfunktionalen Aminocarbonsäuren abgeleitet sind, die nach Einbau in den Linker noch zumindest eine hydrophile Gruppe enthalten.

20. Verwendung gemäß Anspruch 17, wobei die Verzweigungspositionen von polyfunktionellen Aminocarbonsäuren abgeleitet sind.

21. Verwendung gemäß Anspruch 18, 19 oder 20, wobei die polyfunktionellen Aminocarbonsäuren ausgewählt sind aus Lysin, Ornithin, Hydroxylysin, Aspartansäure, Glutaminsäure, Asparagin, Glutamin, Phosphoserin und synthetischen trifunktionellen Aminocarbonsäuren.

22. Verbindung der allgemeinen Formel (I):
Zn- Y - Xm
wobei Z zumindest eine reaktive funktionelle Gruppe oder eine Bindungsgruppe bezeichnet und X zumindest eine reaktive funktionelle Gruppe ist, die über einen Linker Y kovalent an Z gebunden ist, wobei der Linker ein verzweigter Linker ist, der ein Molekulargewicht von > 1.000 Da aufweist und zumindest einen Ladungsträger enthält, n eine Ganzzahl von 1 bis 10 und m 1 oder 2 ist.

23. Verbindung der allgemeinen Formel (I):
Zn- Y - Xm
wobei Z zumindest eine reaktive funktionelle Gruppe oder eine Bindungsgruppe bezeichnet und X zumindest eine reaktive funktionelle Gruppe ist, die über einen Linker Y kovalent an Z gebunden ist, wobei der Linker ein verzweigter Linker ist, der ein Molekulargewicht von > 1.000 Da aufweist und zumindest einen Ladungsträger und zumindest eine hydrophile Gruppe enthält, n eine Ganzzahl von 1 bis 10 und m 1 oder 2 ist.

24. Konjugat, umfassend zumindest eine biologische Substanz und zumindest eine Verbindung der allgemeinen Formel (I) gemäß den Ansprüchen 22 und 23.

25. Konjugat gemäß Anspruch 24, wobei die biologische Substanz ein Antikörper oder Antikörperfragment, eine Nukleinsäure, ein Polypeptidantigen, ein immunologisch reaktives Peptid oder ein Hapten ist.

26. Verwendung von Verbindungen gemäß Anspruch 22 oder 23 oder den Konjugaten gemäß Anspruch 24 oder 25 in einem immunologischen Detektionsverfahren oder in einem Nukleinsäurehybridisierungsverfahren.

27. Verwendung gemäß Anspruch 26 in einem Lumineszenzverfahren.

28. Verwendung gemäß Anspruch 26 oder 27 in einem Elektrochemilumineszenzverfahren.

29. Verwendung gemäß Anspruch 28 zur Verbesserung der Löslichkeit von Markierungsgruppen oder Effektorgruppen oder von ihren Konjugaten.

## Revendications

1. Utilisation d'un composé de formule générale (I) :
Zn - Y - Xm (I)
dans laquelle Z désigne au moins un groupe fonctionnel réactif ou un groupe de liaison et X représente un groupe fonctionnel réactif qui est lié de manière covalente à Z par l'intermédiaire d'un segment de liaison Y, ledit segment de liaison étant un segment de liaison ramifié qui a un poids moléculaire > 1000 Da et contient au moins un support de charge, n représente un nombre entier compris entre 1 et 10 et m vaut 1 ou 2, pour la production de conjugués.

2. Utilisation d'un composé de formule générale (I) :
Zn - Y - Xm (I)
dans laquelle Z désigne au moins un groupe fonctionnel réactif ou un groupe de liaison et X représente un groupe fonctionnel réactif qui est lié de manière covalente à Z par l'intermédiaire d'un segment de liaison Y, ledit segment de liaison étant un segment de liaison ramifié qui a un poids moléculaire > 1000 Da et contient au moins un support de charge et au moins un groupe hydrophile, n représente un nombre entier compris entre 1 et 10 et m vaut 1 ou 2, pour la production de conjugués.

3. Utilisation selon la revendication 1 ou 2, dans laquelle le groupe de liaison est un groupe de marquage ou un groupe effecteur.

4. Utilisation selon l'une quelconque des revendications 1 à 3, dans laquelle le groupe de liaison est un groupe de marquage choisi parmi des groupes de détection luminescents et fluorescents, des enzymes, des microparticules ou des nanoparticules et des radio-isotopes.

5. Utilisation selon la revendication 4, dans laquelle le groupe de marquage est un complexe de chélation métallique et le composé est représenté par la formule générale (II) :
[M(L1L2L3)]n - Y - XmA (II)
dans laquelle
- M représente un cation de métal divalent ou trivalent choisi parmi des ions des métaux de transition ou des terres rares,
- L1, L2 et L3 sont identiques ou différents et désignent des ligands contenant au moins deux hétérocycles contenant de l'azote, L1, L2 et L3 étant liés au cation métallique par l'intermédiaire d'atomes d'azote,
- X représente un groupe fonctionnel réactif qui est lié de manière covalente à au moins l'un des ligands L1, L2 et L3 par l'intermédiaire du segment de liaison ramifié Y,
- n représente un nombre entier compris entre 1 et 10,
- m vaut 1 ou 2, et
- A désigne l'ion complémentaire qui peut être requis pour égaliser la charge.

6. Utilisation selon l'une des revendications 1 à 5, dans laquelle le groupe de liaison est un groupe effecteur choisi parmi des partenaires de paires de liaison de bioaffinité spécifique.

7. Utilisation selon la revendication 6, dans laquelle le groupe effecteur est choisi parmi la biotine et ses analogues, la streptavidine, l'avidine, des antigènes, des haptènes, des anticorps, des acides nucléiques, des analogues d'acide nucléique, des sucres, des lectines, des récepteurs et des ligands de récepteurs.

8. Utilisation selon l'une des revendications 1 à 7, dans laquelle le groupe fonctionnel réactif représente un acide carboxylique, un halogénure d'acide carboxylique, un anhydride d'acide carboxylique, un hydrazide d'acide carboxylique, un azide d'acide carboxylique, une amine, un ester actif, un maléimide, un thiol ou un groupe photoactivable.

9. Utilisation selon l'une des revendications 1 à 8, dans laquelle le segment de liaison ramifié contient au moins un support de charge négative choisi dans le groupe constitué des groupes phosphate, phosphonate, sulfinate, sulfonate, sulfate et carboxylate.

10. Utilisation selon la revendication 9, dans laquelle le segment de liaison ramifié contient au moins un groupe carboxylate et/ou un groupe phosphate.

11. Utilisation selon l'une des revendications 1 à 10, dans laquelle le segment de liaison ramifié contient au moins un support de charge positive choisi parmi des groupes aminés et des groupes aminés substitués.

12. Utilisation selon l'une des revendications 1 à 11, dans laquelle le segment de liaison ramifié contient jusqu'à 70 supports de charge.

13. Utilisation selon la revendication 12, dans laquelle le segment de liaison ramifié contient 1 à 40 supports de charge.

14. Utilisation selon l'une des revendications précédentes, dans laquelle le segment de liaison ramifié contient au moins un groupe hydrophile non chargé choisi parmi les groupes oxyde d'éthylène, oxyde de polyéthylène, sulfoxyde, sulfone, amide d'acide carboxylique, ester d'acide carboxylique, amide d'acide phosphonique, ester d'acide phosphonique, amide d'acide phosphorique, ester d'acide phosphorique, amide d'acide sulfonique, ester d'acide sulfonique, amide d'acide sulfurique et ester d'acide sulfurique.

15. Utilisation selon la revendication 14, dans laquelle au moins un groupe hydrophile non chargé est un groupe amide d'acide carboxylique primaire.

16. Utilisation selon l'une des revendications précédentes, dans laquelle le poids moléculaire du segment de liaison se situe dans la plage comprise entre 1000 et 50 000 Da.

17. Utilisation selon l'une des revendications 1 à 16, dans laquelle le segment de liaison ramifié est au moins partiellement composé d'unités d'acide aminocarboxylique qui sont liées les unes aux autres par des liaisons peptidiques.

18. Utilisation selon la revendication 17, dans laquelle les supports de charge sont dérivés d'acides aminocarboxyliques polyfonctionnels qui contiennent encore au moins un support de charge libre après incorporation dans le segment de liaison.

19. Utilisation selon la revendication 17, dans laquelle les groupes hydrophiles sont dérivés d'acides aminocarboxyliques polyfonctionnels qui contiennent encore au moins un groupe hydrophile après incorporation dans le segment de liaison.

20. Utilisation selon la revendication 17, dans laquelle les points de ramification sont dérivés d'acides aminocarboxyliques polyfonctionnels.

21. Utilisation selon la revendication 18, 19 ou 20, dans laquelle les acides aminocarboxyliques polyfonctionnels sont choisis parmi la lysine, l'ornithine, l'hydroxylysine, l'acide aspartique, l'acide glutamique, l'asparagine, la glutamine, la phosphosérine et des acides aminocarboxyliques trifonctionnels de synthèse.

22. Composé de formule générale (I)
Zn - Y - Xm
dans laquelle Z désigne au moins un groupe fonctionnel réactif ou un groupe de liaison et X représente au moins un groupe fonctionnel réactif qui est lié de manière covalente à Z par l'intermédiaire d'un segment de liaison Y, où le segment de liaison est un segment de liaison ramifié qui a un poids moléculaire > 1000 Da et contient au moins un support de charge, n représente un nombre entier compris entre 1 et 10 et m vaut 1 ou 2.

23. Composés de formule générale (I)
Zn - Y - Xm
dans laquelle Z désigne au moins un groupe fonctionnel réactif ou un groupe de liaison et X représente au moins un groupe fonctionnel réactif qui est lié de manière covalente à Z par l'intermédiaire d'un segment de liaison Y, où le segment de liaison est un segment de liaison ramifié qui a un poids moléculaire > 1000 Da et contient au moins un support de charge et au moins un groupe hydrophile, n représente un nombre entier compris entre 1 et 10 et m vaut 1 ou 2.

24. Conjugué comprenant au moins une substance biologique et au moins un composé de formule générale (I) selon les revendications 22 et 23.

25. Conjugué selon la revendication 24, dans lequel la substance biologique est un anticorps ou un fragment d'anticorps, un acide nucléique, un antigène polypeptidique, un peptide immunologiquement réactif ou un haptène.

26. Utilisation de composés selon les revendications 22 ou 23 ou des conjugués selon les revendications 24 ou 25 dans un procédé de détection immunologique ou dans un procédé d'hybridation d'acide nucléique.

27. Utilisation selon la revendication 26 dans un procédé de luminescence.

28. Utilisation selon la revendication 26 ou 27 dans un procédé d'électrochimiluminescence.

29. Utilisation selon la revendication 28 pour améliorer la solubilité de groupes de marquage ou de groupes effecteurs ou de leurs conjugués.
